(19) Europäisches Patentamt
European Patent Office
Office européen des brevets

(11) **EP 4 215 183 A1**

(12) **EUROPEAN PATENT APPLICATION**
published in accordance with Art. 153(4) EPC

(43) Date of publication:
**26.07.2023 Bulletin 2023/30**

(21) Application number: **21869412.3**

(22) Date of filing: **16.09.2021**

(51) International Patent Classification (IPC):
*A61K 9/08* (2006.01)    *A61B 1/00* (2006.01)
*A61K 47/02* (2006.01)    *A61K 47/22* (2006.01)
*A61K 49/00* (2006.01)    *A61K 49/04* (2006.01)
*A61K 38/48* (2006.01)

(52) Cooperative Patent Classification (CPC):
**A61B 1/00; A61K 9/08; A61K 38/48; A61K 47/02; A61K 47/22; A61K 49/00; A61K 49/04**

(86) International application number:
**PCT/JP2021/034040**

(87) International publication number:
**WO 2022/059728 (24.03.2022 Gazette 2022/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.09.2020  JP 2020156704**

(71) Applicants:
• **Kaken Pharmaceutical Co., Ltd.**
**Tokyo 113-8650 (JP)**
• **Japanese Foundation For Cancer Research**
**Tokyo 135-8550 (JP)**

(72) Inventor: **HORIUCHI Yusuke**
**Tokyo 135-8550 (JP)**

(74) Representative: **Dr. Schön, Neymeyr & Partner Patentanwälte mbB**
**Bavariaring 26**
**80336 Munich (DE)**

(54) **DIAGNOSIS ACCURACY IMPROVING COMPOSITION FOR USE IN MAGNIFICATION ENDOSCOPY OR ULTRA-HIGH MAGNIFICATION ENDOSCOPY**

(57)    For diagnosing gastric cancer by magnifying endoscopy with narrow-band imaging or endocytoscopy with narrow-band imaging where light of narrow-band width is used for observation, a high-performance diagnostic method was established. In place of staining with a dye conducted in other organs, pronase is orally administered prior to endoscopic examination and topically administered under endoscopic observation as needed. In this manner, endoscopy can be performed without promoting production of mucosal fluid. In conventional endoscopic examination, a lesion is found and definitive diagnosis is made based on pathological examination. In contrast, this method can provide high diagnostic performance, suggesting that accurate diagnosis equivalent to that provided by pathological examination can be made by endoscopy. Removal of mucosal fluid with pronase is useful in magnifying endoscopy and endocytoscopy.

FIG.4

EP 4 215 183 A1

**Description**

[Technical Field]

The present invention relates to a composition responsible for accuracy of diagnosis for gastric cancer in magnifying endoscopy and endocytoscopy, particularly magnifying endoscopy with narrow-band imaging and endocytoscopy with narrow-band imaging, in which observation is made under narrow-band light in addition to observation under ordinary light, an observation kit and methods for applying thereof.

[Background Art]

Gastric cancer is one of cancers having not only high morbidity but also high mortality. With recent advancements of endoscopic instruments, the number of gastric cancer cases found in early stages has increased and mortality thereof has decreased. When gastric cancer in early stage, surgical resection of the cancer can be avoided by using ESD (endoscopic submucosal dissection) to save the stomach. Endoscopy contributes to not only diagnosis but also therapy.
A tumor developed in the gastrointestinal tract has been observed by endoscopy under irradiation of ordinary light (white light). Not only the presence of a tumor can be diagnosed but also qualitative diagnosis for distinguishing a tumor from a non-tumor and quantitative diagnosis for determining the depth/range of a tumor, have been made. Recently, qualitative diagnosis and quantitative diagnosis (depth/range) have been more accurately made by using NBI (Narrow Band Imaging (registered trademark)).
NBI is a technique where two light beams different in wavelength, which are easily absorbed by blood hemoglobin and brought into a narrow band, are applied to enhance display of blood capillaries and mucosal micropattern on mucosal surface. In a cancerous area blood flows differently from the ambient region. Because of this, if NBI is used, cancer can be accurately diagnosed. It has been reported that if magnifying endoscopy with narrow-band imaging (ME-NBI) is used, diagnostic accuracy of cancer can be improved. ME-NBI is presently a standard examination for gastric cancer (Non Patent Literatures 1 and 2).
Further recently, endocytoscopy (EC) has been developed. The magnifying power of the conventional magnifying endoscopy fall within the range of about 80 to 100 times, whereas that of EC can fall within the range of about 400 to 500 times. Since the magnifying power of EC is equivalent to that of microscopy used for pathological diagnosis, morphological observation of cells and nuclei can be made by staining with methylene blue/crystal violet. Owing to EC, diagnosis can be made without biopsy. Usefulness of EC has been reported for diagnosis of esophageal cancer and colon cancer (Non
Patent Literatures 3 to 7). It has been also reported that when EC is used in combination with narrow-band imaging (EC-NBI) for diagnosing colon cancer, diagnostic performance of EC-NBI significantly increases beyond conventional ME-NBI (Non Patent Literature 8). Although there are reports that gastric cancer was diagnosed by endocytoscopy in combination with staining, a large amount of mucosal fluid was produced in the stomach and prevented uniform staining. A long time is required for staining and staining further stimulates/promotes production of mucosal fluid, with the result that a lesion becomes obscure. These are problems so far pointed out (Non Patent Literatures 9 and 10).

[Citation List]

[Non Patent Literatures]

[Non Patent Literature 1] Ezoe Y., et al., 2011, Gastroenterology, Vol. 141, pp. 2017-2025.
[Non Patent Literature 2] Yao K. et al., 2009, Endoscopy, Vol. 41, pp. 462-467.
[Non Patent Literature 3] Kumagai Y. et al., 2004, Endoscopy. Vol. 36, pp. 590-594.
[Non Patent Literature 4] Inoue H., et al., 2004, Gastrointest Endosc. Clin. N. Am., Vol. 14, pp. 589-594.
[Non Patent Literature 5] Kumagai Y., et al., 2017, Endoscopy, Vol. 49, pp. 176-180.
[Non Patent Literature 6] Sasajima K., et al., 2006, Gastrointest. Endosc. Vol. 63, pp. 1010-1017.
[Non Patent Literature 7] Sako T., et al., 2018, Endoscopy, Vol. 50, pp. 69-74.
[Non Patent Literature 8] Kudo S.E. et al., 2015, Gastrointest. Endosc. Vol. 82, pp. 912-923.
[Non Patent Literature 9] Kaise M. et al., 2015, Endoscopy, Vol. 47, pp. 19-25.
[Non Patent Literature 10] Abad M.R.A., et al., 2020, Transl. Gastroenterol. Hepatol., 5: 28. doi: 10.21037/tgh.2019.11.12
[Non Patent Literature 11] Muto M. et al., 2016, Dig. Endosc. Vol. 28, pp. 379-393.

[Non Patent Literature 12] Nakanishi H. et al., 2017, Endoscopy. Vol. 49, pp. 957-67.

[Summary of Invention]

[Technical Problem]

With the advancement of endoscopic instruments as mentioned above, diagnostic methods for gastrointestinal cancer have changed. Particularly, with the development of EC, endoscopic diagnosis has qualitatively changed. In colon cancer, since morphological observation of nuclei and cells can be made by staining, diagnosis can be made with the same accuracy as in cytology, realizing definitive diagnosis. In other words, conventionally, a lesion was found by endoscopy and definitive diagnosis was made based on pathological examination, but now, definitive diagnosis can be made by endoscopy. However, a suitable examination method varies depending on the organ. The diagnostic method already established for colon cancer may not always bring appropriate results for gastric cancer diagnosis. As mentioned above, when the staining, which contributes to highly accurate diagnosis of colon cancer, is used for diagnosis of gastric cancer, uniform staining cannot be made due to the mucosal fluid, with the result that a lesion becomes obscure. So, it is difficult to confirm diagnosis by EC. The present inventor studied a method for diagnosing gastric cancer by EC-NBI and established a method for more accurately diagnosing gastric cancer than the examination method using ME-NBI presently employed as a standard examination. He investigated a pretreatment method and composition required for improving diagnostic accuracy and accomplished the present invention.

In diagnosing gastric cancer, if EC-NBI is more useful than ME-NBI, there is a possibility that EC-NBI may be used in place of ME-NBI as a standard examination. Change of a standard examination to EC-NBI means that accurate diagnosis equivalent to that provided by pathological examination can be made by endoscopy. The present invention relates to endoscopic examination providing highly accurate diagnosis of gastric cancer, that is, an examination method providing accurate diagnosis of gastric cancer equivalent to diagnosis of colon cancer and esophageal cancer obtained by EC. In colon cancer and esophageal cancer, morphological observation of cells and nuclei is made by EC in combination with staining with e.g., methylene blue. However, if staining is applied to gastric cancer, the image of a lesion becomes obscure and cannot be observed by EC. Then, the present inventor investigated establishment of the most suitable method for diagnosing gastric cancer by using EC in combination with NBI (EC-NBI). As a result, he found that a lesion image becomes obscure by mucosal fluid secreted from the stomach in ME-NBI; and that the same applies to EC-NBI. Thus, an object of the present invention is to provide a pretreatment method and composition for making gastric cancer diagnosis without being influenced by the mucosal fluid. Further, he clarified difference in diagnostic performance between EC-NBI and ME-NBI (as a standard examination) to show the possibility of improving diagnostic accuracy of gastric cancer in daily medical practice. The usefulness of EC-NBI, if it is clearly confirmed, can lead to establishment of the most suitable diagnostic method for gastric cancer and provide a new therapeutic method. An object of the invention of the present application is to provide magnifying endoscopic examination, in particular, a composition and an observation kit required for diagnosis by EC-NBI.

[Solution to Problem]

The present invention relates to the following composition, kit and examination method for improving diagnostic accuracy.

(1) A composition for improving diagnostic accuracy for use in improving diagnostic accuracy of gastroendoscopy, containing a proteolytic enzyme for use in magnifying endoscopy or endocytoscopy, as an active ingredient.
(2) The composition for improving diagnostic accuracy according to (1), wherein the proteolytic enzyme is pronase.
(3) The composition for improving diagnostic accuracy according to (1) or (2), which is administered in advance of magnifying endoscopic examination or endocytoscopic examination.
(4) The composition for improving diagnostic accuracy according to any one of (1) to (3), which is administered in advance of magnifying endoscopic examination or endocytoscopic examination and also supplementarily administered.
(5) The composition for improving diagnostic accuracy according to (3) or (4), wherein the composition for improving diagnostic accuracy to be administered in advance is a solution for oral administration prepared by dissolving 20,000 units of pronase and 1 g of sodium hydrogen carbonate in 50 to 80 mL of water or a solution for oral administration prepared by dissolving 20,000 units of pronase in 50 to 80 mL of alkaline ionized water.
(6) The composition for improving diagnostic accuracy according to (4), wherein the composition for improving diagnostic accuracy to be supplementarily administered is a pronase solution prepared by dissolving 20,000 units of pronase and 1 g of sodium hydrogen carbonate in 2 to 60 mL of water or a pronase solution prepared by dissolving 20,000 units of pronase in 2 to 60 mL of alkaline ionized water.

(7) The composition for improving diagnostic accuracy according to (4) or (6), wherein the composition for improving diagnostic accuracy to be supplementarily administered is provided in the form of a double-chamber prefilled syringe consisting of one chamber filled with pronase and sodium hydrogen carbonate and the other chamber filled with water or consisting of one chamber filled with pronase and the other chamber filled with alkaline ionized water, and dissolved just before using.

(8) The composition for improving diagnostic accuracy according to any one of (4), (6) or (7), wherein the supplementary administration is topically administration to be performed under endoscopic observation.

(9) The composition for improving diagnostic accuracy according to any one of (1) to (8), wherein the magnifying endoscopy or endocytoscopy is endoscopy with narrow-band imaging.

(10) A mucosal membrane observation kit containing pronase, sodium hydrogen carbonate and/or alkaline ionized water for endoscopic examination.

(11) The mucosal membrane observation kit for supplementary administration according to (10), wherein the kit is provided in the form of a double-chamber prefilled syringe consisting of one chamber filled with pronase and sodium hydrogen carbonate and the other chamber filled with water or consisting of one chamber filled with pronase and the other chamber filled with alkaline ionized water; and pronase and water or alkaline ionized water are filled such that a concentration of pronase is 333 units/ml or more and 10,000 units/ml or less.

(12) An upper gastrointestinal endoscopic examination method comprising orally administering a composition for improving diagnostic accuracy containing a proteolytic enzyme as an active ingredient before endoscopy and conducting examination by magnifying endoscopy or endocytoscopy.

(13) The upper gastrointestinal endoscopic examination method according to (12), wherein the proteolytic enzyme is pronase.

(14) The upper gastrointestinal endoscopic examination method according to (12) or (13), wherein the oral administration is made 15 to 30 minutes before initiation of upper gastrointestinal endoscopic examination.

(15) The upper gastrointestinal endoscopic examination method according to (13) or (14), wherein the pronase to be orally administered is provided in the form of a solution prepared by dissolving 20,000 units of pronase and 1 g of sodium hydrogen carbonate in 50 to 80 mL of water or dissolving 20,000 units of pronase in 50 to 80 mL of alkaline ionized water.

(16) The upper gastrointestinal endoscopic examination method according to any one of (12) to (15), further comprising supplementarily administering a composition for improving diagnostic accuracy to a surface or interface part of a lesion under endoscopic observation.

(17) The upper gastrointestinal endoscopic examination method according to (16), wherein the supplementary administration of a composition for improving diagnostic accuracy is administration via a syringe through a forceps channel of an endoscope or spraying via a spray tube inserted through the forceps channel.

(18) The upper gastrointestinal endoscopic examination method according to (16) or (17), wherein pronase to be supplementarily administered is provided in the form of a solution prepared by dissolving 20,000 units of pronase and 1 g of sodium hydrogen carbonate in 2 to 60 mL of water or 20,000 units of pronase in 2 to 60 mL of alkaline ionized water.

(19) The upper gastrointestinal endoscopic examination method according to any one of (12) to (18), to be performed without staining.

(20) The upper gastrointestinal endoscopic examination method according to any one of (12) to (19), comprising taking a cytology sample from a body site under observation.

(21) A therapeutic method comprising performing the upper gastrointestinal endoscopic examination method according to any one of (12) to (20) and resecting an abnormal site if found by endoscopic submucosal dissection.

(22) The upper gastrointestinal endoscopic examination method according to any one of (12) to (20) or the therapeutic method according to (21), wherein the magnifying endoscopy or endocytoscopy is endoscopy with narrow-band imaging.

(23) A diagnostic method for gastric cancer by endocytoscopy with narrow-band imaging, comprising orally administering the composition for improving diagnostic accuracy according to any one of (1) to (5) before the endoscopy, making observation by endocytoscopy with narrow-band imaging and making a determination.

(24) The diagnostic method for gastric cancer according to (23), comprising administering the composition for improving diagnostic accuracy according to (6) to a surface or interface part of a lesion under endoscopic observation., followed by observing.

[Brief Description of Drawings]

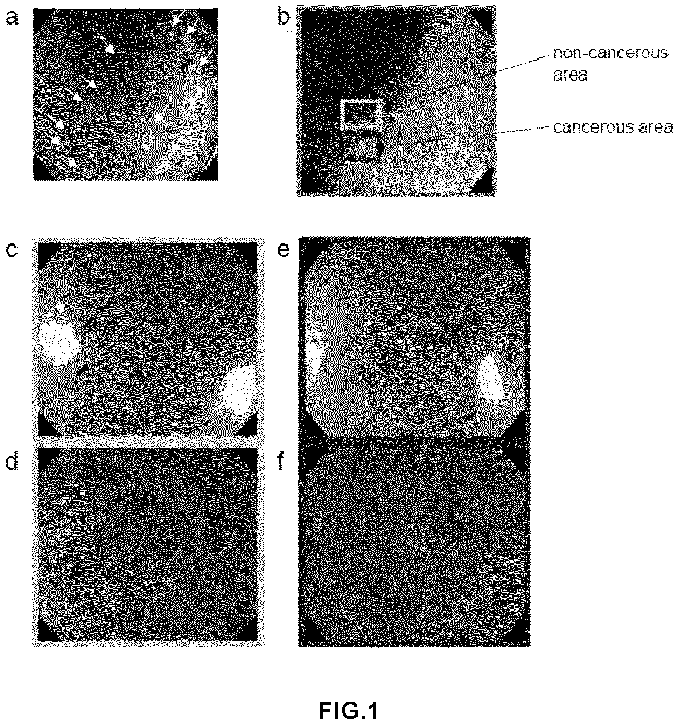

[Figure 1] The figure shows images of cancerous area and non-cancerous area of the stomach observed by ME-NBI and EC-NBI. Figures d and f show images observed by EC-NBI newly set as a standard by the present invention. Figure a shows a white light image. Perilesional sites were marked (indicated by white arrows) before ESD. The square frame shows an interface part between a cancerous area and a non-cancerous area of a lesion located closest to the mouth.

Figure b shows an image of the interface part between a cancerous area and a non-cancerous area of a lesion located closest to the mouth observed by non-magnifying endoscopy with NBI. A non-cancerous area and a cancerous area are separately surrounded with a square frame.

Figure c shows an image of a non-cancerous area observed by ME-NBI. No irregularity is found in either blood vessel or surface structure.

Figure d shows an image of a non-cancerous area observed by EC-NBI (the same site observed in the ME-NBI image). No irregularity is found in either blood vessel or surface structure. The same site as in Figure c is enlarged about 500 times the original size.

Figure e shows an image of a cancerous area observed by ME-NBI. Irregularity of different diameters is observed in blood vessel from the center toward the lower left.

Figure f shows an image of a cancerous area observed by EC-NBI (the same site observed in the ME-NBI image). Non-uniform tortuous blood vessel varying throughout in diameter and partially expanded is observed. Irregularity in blood vessel is observed. The same site as in Figure e is enlarged about 500 times the original size.

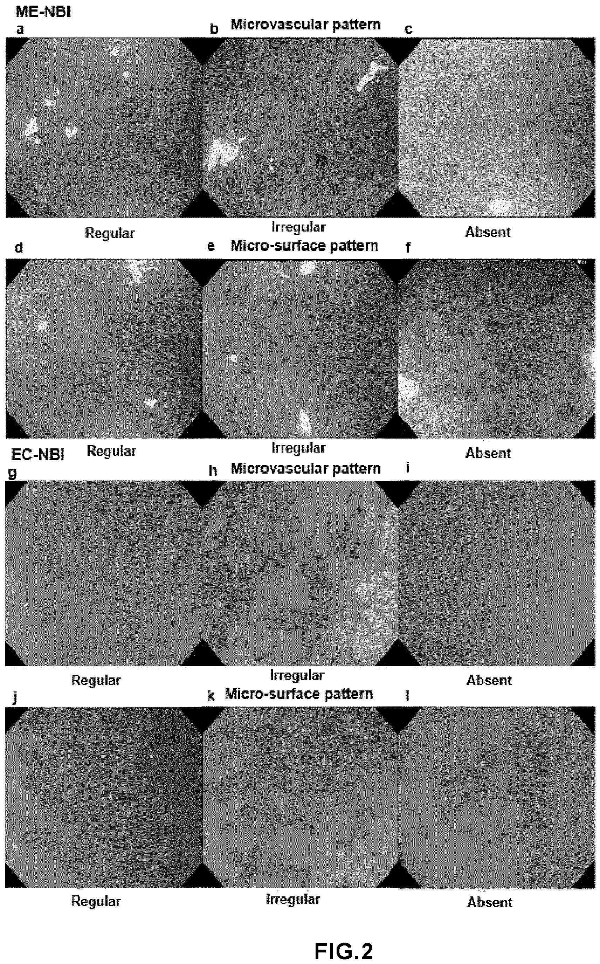

[Figure 2] The figure shows VS (Vessel Plus Surface) classification in ME-NBI and EC-NBI. Figures g to l are images of vessels and surface structures newly set by application of VS classification. ME-NBI

Micro-vascular pattern

Figure a. Regular: uniform net-like blood vessels are observed
Figure b. Irregular: blood vessels that expand locally and have different caliber meander
Figure c. Absent: no vascular findings (Absent) Micro-surface pattern
Figure d. Regular: uniform surface structure is observed
Figure e. Irregular: non-uniform surface structures are observed
Figure f. Absent: no surface structures are observed EC-NBI

Micro-vascular pattern

Figure g. Regular: uniform blood vessels in shape are observed
Figure h. Irregular: blood vessels that expand locally and have different in diameter caliber meander
Figure I. Absent: no vascular finding Micro-surface pattern
Figure j. Regular: uniform surface structure is observed
Figure k. Irregular: non-uniform surface structures are observed
Figure l. Absent: no surface structures are observed
[Figure 3] The figure shows cases and lesions in images used for evaluation.
[Figure 4] The figure shows comparison in diagnostic accuracy, sensitivity and specificity between ME-NBI and EC-NBI performed by all endoscopists.
[Figure 5] The figure shows comparison in positive predictive value and negative predictive value between ME-NBI and EC-NBI performed by all endoscopists.
[Figure 6] The figure shows cases of gastric cancer subjected to EC with staining.

[Description of Embodiments]

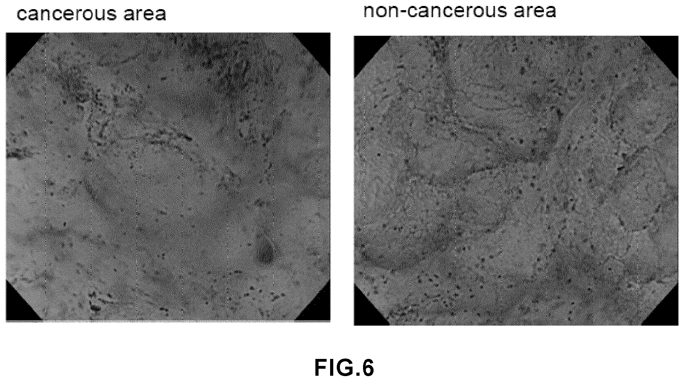

In diagnosis of colon cancer, morphological observation of cells and nuclei can be made by staining them with methylene blue/crystal violet. Since it is possible to obtain analogous images obtained by conventional cytology, diagnostic performance by EC is reported to be extremely high. However, in gastric cancer, when the same staining as in colon cancer is carried out, mucosal fluid production is stimulated/promoted by staining, with the result that a lesion becomes obscure, as described above. An image of gastric cancer obtained by EC in combination with staining is shown as a reference example in Figure 6. In this case, pronase is orally taken before endoscopic observation. Then, a site of interest is stained with crystal violet and methylene blue. After staining, pronase is further sprayed and an image is photographed. As shown in Figure 6, the image is obscure and a cancerous area and a non-cancerous area cannot be distinguished. In other words, gastric cancer cannot be examined by staining in the same manner as in colon cancer. In the circumstance, the present inventor investigated a method having high diagnostic performance without promoting mucosal fluid production.

As described below, endoscopic images were checked by many doctors belonging to different facilities and having different experiences and determined whether highly accurate diagnosis can be made or not. It was found that it is important to obtain a clear image for diagnostic accuracy as described later; and that, for obtaining a clear image, mucosal fluid produced from the stomach have to be removed. The present invention relates to a method for removing mucosal fluid for attaining high diagnostic accuracy and a pharmaceutical composition for removing mucosal fluid.

The magnifying endoscopy used herein refers to an endoscopy that can magnify an object on the monitor up to about 100 times, whereas the endocytoscopy (micro-endoscopy) refers to an endoscopy that can magnify an object up to 500 times or more owing to optical function of magnification. Magnifying endoscopy and endocytoscopy can be preferably used together with narrow-band imaging. In the first place, we studied about diagnostic accuracy using endoscopy and details of the study will be described below.

[Study method]

ESD was continuously carried out by a physician from November, 2016 to July, 2019. Images and information of the case were extracted from electronic medical charts and the images were used as an evaluation target. Note that, investigation/analysis was conducted after an approval by the Independent Ethics Committee (IRB) was obtained and in accordance with the Helsinki Declaration and the following revised rules. In recording the research data, personal identity information was all deleted. Informed consent on use of pathological specimens and image data for the purpose of study was obtained from each of the patients.

An ME-NBI image is taken in the period of detailed examination before a therapy, whereas an EC-NBI image is taken in the period of marking for ESD. The ME-NBI image and EC-NBI image are both extracted from the images of a gastric cancerous area located closest to the mouth and the adjacent non-cancerous area. The ME-NBI image and EC-NBI image are images of the same site in the same case. Images actually photographed are shown in Figure 1. Note that cancerous areas and non-cancerous areas in all cases were subjected to ESD and then pathological examination, and confirmed in accordance with "golden standard", Gastric Cancer treatment guidelines (5th version edited by the Japanese Gastric Cancer Association).

[Criteria of images]

Criteria for eligibility

    1. ME-NBI image and EC-NBI image of the same case are both photographed.
    2. Both in ME-NBI and EC-NBI, a gastric cancerous area located closest to the mouth and the adjacent non-cancerous area are observed by magnification.

Exclusion criteria

    1. Case where magnifying endoscopic observation by either ME-NBI or EC-NBI failed
    2. Case where an image is obscure due to, e.g., mucosal fluid, blood, halation

All images are selected by supervisory doctor belonging to the Japan Gastroenterological Endoscopy Society and further confirmed by another supervisory doctor belonging to the Japan Gastroenterological Endoscopy Society as to whether they satisfy the criterion for eligibility and the exclusion criterion. The endoscopes used for ME-NBI are GIF-H260Z, GIF-H290Z; and those for EC-NBI are GIF-Y0002, GIF-H290EC (all are manufactured by OLYMPUS Medical Systems).

[Flow of endoscopic procedure]

An ME-NBI image is photographed during detailed examination before a therapy. An endoscope is inserted after the tip was covered with black hood (MB-46 (Olympus Medical Systems)). After observation under ordinary light is carried out, the range to be diagnosed is determined by ME-NBI. Then, a cancerous area and a non-cancerous area are observed by magnification, and then, subjected to chromoendoscopic observation.

An EC-NBI image is photographed during ESD. An endoscope is inserted without hood. EC-NBI is adjusted and the range to be diagnosed is determined at a comparable magnification that of ME-NBI. Marking is carried out, and thereafter, a cancerous area and a non-cancerous area are observed by ultra-high magnification with NBI.

[Endoscopist evaluating image and evaluation method]

Contacts were made with endoscopists (contact addresses were known) belonging to institutions in Japan and asked whether they would participate in a diagnostic study for cancer/non-cancer based on ME-NBI image and EC-NBI image. Endoscopists who gave informed consent on participation were employed as subjects. As a diagnostic method, the diagnostic method for ME-NBI using VS (Vessel Plus Surface) classification (Non Patent Literatures 2 and 11) proposed by the Japan Gastroenterological Endoscopy Society as diagnostic guidelines was employed. VS classification is a classification for distinguishing cancer/non-cancer by ME-NBI, and consists of vascular observations (V) and surface structure observations (S). If any irregularity is found in both observations (V and S), the case is diagnosed as cancer. In the invention, the VS classification was employed for evaluation by not only ME-NBI but also EC-NBI.

Before diagnosis, typical images of sites corresponding to "regular", "irregular" and "absent" (Figure 2) observed in ME-NBI and EC-NBI were delivered to endoscopists. Since there were no reports on diagnostic method based on images obtained by EC-NBI as described above, diagnosis was made in accordance with the diagnostic method for ME-NBI. Diagnosis is made in accordance with the following definitions: If an irregularity is found in either microvascular observation or micro-surface observation, the case is defined as cancer and the other cases are defined as non-cancer. Based on understanding of the content of the study, the endoscopists made diagnosis (gastric cancer, non-cancer or unclear) with reference to images provided by ME-NBI and EC-NBI (with respect to gastric cancer and non-cancer). Note that we did not inform to diagnosticians (endoscopists) in diagnosis that the same case was employed by ME-NBI and EC-NBI, and that a gastric cancerous area located closest to the mouth and the adjacent non-cancerous area were photographed. As information about the diagnosticians (endoscopists), we collected the total number of endoscopies so far performed, years of experience as an endoscopist, possession of a license as a specialist approved by the Japan Gastroenterological Endoscopy Society, training history in a specialized facility for ME-NBI, and years of experience for ME-NBI and EC.

[Criteria for evaluation]

Evaluation was made based on the Standards for the Reporting of Diagnostic Accuracy Studies 2015 guidelines. Diagnostic accuracy, sensitivity and specificity for each of ME-NBI images and EC-NBI images were evaluated.

Diagnostic accuracy is defined as follows:

```
(Number of images correctly diagnosed as cancer +

number of images correctly diagnosed as non-cancer)/total

number of images
```

A positive predictive value (PPV) and a negative predictive value (NPV) are also evaluated. Based on these, diagnostic performances of ME-NBI and EC-NBI are compared. Since it has been reported that diagnostic performance of an endoscopist based on ME-NBI is improved by receiving training for ME-NBI (Non Patent Literature 12), the endoscopists were evaluated based on training experience in a specialized facility.

[Statistical items]

Endoscopists were divided into groups based on the possession or non-possession of license as a specialist approved by the Japan Gastroenterological Endoscopy Society. The total number of endoscopies performed, years of experience as an endoscopist and years of experience for ME-NBI were separately added up for each group. Thereafter, a cut-off value was set such that the N values of both groups became as close as possible. Two groups, a group of endoscopists having ME-NBI training history and a group of endoscopists having no ME-NBI training history, were subjected to the Fisher test.

With respect to diagnostic performance, a median was set for all parameters, and 95% confidence interval was set as the interquartile range. Comparison was made by the Mann-Whitney U test. Comparison of the endoscopists with respect to background and characteristics and diagnostic performances of all endoscopists based on ME-NBI and EC-NBI were made by setting the level of statistical significance as p < 0.05. When ME-NBI and EC-NBI diagnostic performances of the endoscopists within a group were compared based on the presence or absence of training history of ME-NBI, the Bonferroni method was employed and the level of statistical significance was set as p < 0.05/2. In this manner, two items were compared in the same group. All analyses were made by use of JMP version 13.2 (SAS (registered trademark) Institute, Cary).

[Results]

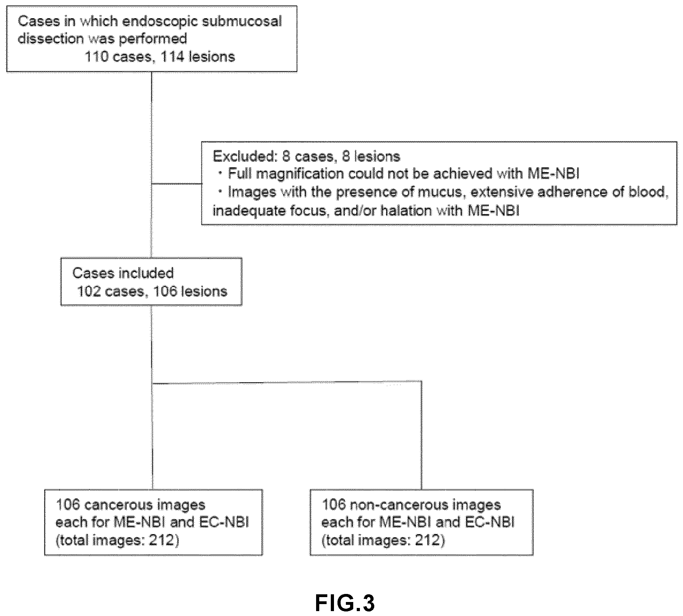

The total number of cases was 110 with 114 lesions. The number of cases satisfying criteria for eligibility was 102 with 106 lesions (Figure 3). Backgrounds of patients and features of lesions are listed in Table 1. Note that the numbers of images of cancerous and non-cancerous areas used in diagnosis by ME-NBI and EC-NBI each were 106 and 424 in total. In the table, the numerical value within parentheses indicates percentage (%). The numerical values in each of the columns of "Age" and "Tumor size" represent median, interquartile range and range in this order from the left.

[Table 1] Patient's background and features of lesion

| Patient's background/lesion | | | 102 cases/ 106 lesions |
|---|---|---|---|
| Age | | | 71(61.8-77) [26-87] |
| Gender (male/female) | | | 66(64.7)/ 36 (35.3) |
| Location | | | |
| | Upper third | | 26(24.5) |
| | Middle third | | 59(55.7) |
| | Lower third | | 17 (16.0) |
| | Gastric tube | | 4(3.8) |
| Macroscopic type | | | |
| | Elevated | | 15(14.2) |
| | Flat | | 5(4.7) |
| | Depressed | | 83 (78.3) |
| | Complex | | 3(2.8) |
| Tumor diameter (mm) | | | 14(9-20.3) [1.5-5.7] |
| Invasion depth | pT1a | | 90 (84.9) |
| | pT1b | <500um | 12(11.3) |
| | | $\geqq 500\mu m$ | 4(3.8) |
| Ulcerative findings | | | |
| | Present | | 5(4.7) |
| | Absent | | 101(95.3) |
| Histological type | | | |
| | Differentiated | | 82(77.4) |
| | Undifferentiated | | 24 (22.6) |

The number of the endoscopists that participated in the study was 61 from 45 institutions. The proportion of the endoscopists having engaged in endoscopy for 10 years or more was 65.6%. The proportion of the endoscopists having 10,000 or more of endoscopic observation cases was 50.8%. The proportion of the endoscopists having a license as a specialist was 68.9%. The proportion of the endoscopists having training history for ME-NBI was 54.1%. The proportion of the endoscopists having at least 5-year experience of ME-NBI was 34.4%. The proportion of the endoscopists having experience of EC was 13.1%.

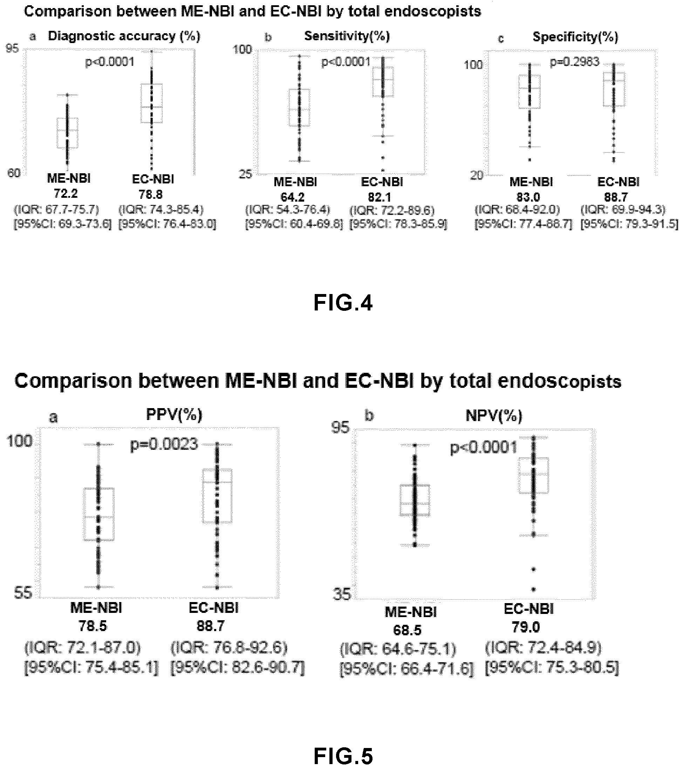

Comparison of diagnostic performances of all participants based on ME-NBI and EC-NBI are shown. Comparison results of diagnostic accuracy, sensitivity and specificity are shown in Figure 4. Comparison results of positive predictive value (PPV) and negative predictive value (NPV) are shown in Figure 5. As a whole, the diagnostic accuracy, sensitivity and NPV of EC-NBI were significantly higher than those of ME-NBI. The diagnostic performances of all participants by EC-NBI were significantly higher than those by ME-NBI (Figure 4). Since endoscopic examination is a screening test, the particularly important item is sensitivity. The sensitivity in EC-NBI was 82.1% (interquartile range:72.2 to 89.6, 95% CI: 78.3 to 85.9), which was higher than 64.2% (interquartile range: 54.3 to 76.4, 95% CI: 60.4 to 69.8) in ME-NBI.

In all participants, the positive predictive value and negative predictive value in EC-NBI were both significantly higher than those in ME-NBI (Figure 5). In particular, the positive predictive value in EC-NBI was 88.7% (interquartile range: 76.8 to 92.6, 95% CI: 82.6 to 90.7), which was higher than 78.5% (interquartile range: 72.1 to 87.0, 95% CI: 75.4 to 85.1) in ME-NBI.

When endoscopists were divided into groups based on the presence or absence of training history for ME-NBI, diagnostic performance including sensitivity by EC-NBI was significantly higher in a group having ME-NBI training history than that by ME-NBI. Also in a group having no ME-NBI training history, diagnostic performance by EC-NBI was significantly higher than that by ME-NBI, similarly to the group having ME-NBI training history (detailed data are not shown). In other word, regardless of training history, diagnostic performance based on EC-NBI images was higher. The reason is conceivable as follows: as shown in Figure 1, images by EC-NBI can be observed at a higher magnification than those by ME-NBI, which means that changes in structure and blood vessel caused by cancer can be more specifically observed, with the result that diagnosis can be highly accurately made. It should be noted that it is important to keep the tissue surface of an area as an observation target clean and transparent in accordance with the present invention (as described later).

The diagnostic performance based on EC-NBI was clearly demonstrated herein. It was also demonstrated that the diagnostic performances of all endoscopists for cancer or non-cancer based on EC-NBI images are higher than those based on ME-NBI images. It was further demonstrated that the diagnostic performances of the endoscopists having no ME-NBI training based on EC-NBI images are also high. The investigation results may change a current diagnostic system for gastric cancer based on ME-NBI and can contribute to improvement of the diagnostic performance for gastric cancer. It is considered that diagnosis of gastric cancer by EC-NBI is beneficial in daily medical practice. In this study, different from previous reports on EC in combination with staining, there were no obscure images due to mucosal fluid in both ME-NBI and EC-NBI and accurate observation were successfully made. Staining in EC has disadvantages since time for staining is required and mucosal fluid is produced. Avoidance of these disadvantages is a merit of EC-NBI. From the results, it was also found that diagnostic method based on ME-NBI is applicable to that based on EC-NBI. From this, it was suggested that diagnosing cancer/non-cancer based on EC-NBI images rather than ME-NBI images is useful in daily medical practice.

Most of the images provided by ME-NBI and EC-NBI used for evaluation herein were clear sufficient to be used for evaluation. When we analyzed why clear images were obtained, it was found that administration of pronase is involved. In the institution to which the present inventor belongs to, when upper gastrointestinal endoscopic examination is conducted, gastric mucosal fluid is dissolved/removed by orally administering a solution, which was prepared by dissolving 20,000 units of pronase and 1 g of sodium hydrogen carbonate in about 50 to 80 mL of water 15 to 30 minutes before the endoscopy. The pronase used herein is pronase MS (manufactured by KAKEN PHARMACEUTICAL CO., LTD.). In the examples subjected, administration was carried out in the same manner. Note that conceivable accidental symptoms caused by administration were not observed. Note that, pronase is used herein but, needless to say, a pharmaceutical composition containing a proteolysis enzyme as an active ingredient can be used as long as the enzyme has the same mucosal fluid removal effect as pronase.

It has been reported that administration of pronase is effective in ordinary endoscopy and chromoendoscopy. However, pronase is not always administered in ordinary endoscopy and chromoendoscopy. In contrast, as shown below, in order to obtain high diagnostic performance in ME-NBI and EC-NBI, it is considered essential to administer pronase before endoscopy. Further, it is important to topically administer pronase as needed, under endoscopic observation, for enhancing diagnostic performance. Since accidental symptoms conceivably caused by administration of pronase were not observed, it is preferable that pronase is used for not only clinical examinations/therapies by ME-NBI and EC-NBI but also ordinary endoscopy and chromoendoscopy.

Pronase is administered when screening test of the entire stomach is conducted by ordinary endoscopy and chromoendoscopy, because mucosal fluid has to be removed from the entire stomach. For the purpose, it is desirable to provide pronase uniformly within the stomach by orally taking a pronase solution prepared by dissolving pronase in about 50-80 ml of water. In the cases of ME-NBI and EC-NBI, although it is important to remove mucosal fluid from the entire stomach, mucosal fluid is removed primarily for observing a lesion. Thus, it is necessary to remove mucosal fluid from a surface or interface part of a lesion, administration of pronase with pinpoint accuracy is required. If administration is required under endoscopic observation, pronase is administered through a forceps channel of an endoscope by inserting a syringe filled with pronase in the hole or supplementarily administered through a spray tube by inserting it through a forceps channel and placing it right above a lesion. The concentration of pronase to be supplementary administered is sometimes set higher than that orally administered. More specifically, an aqueous solution containing 20,000 units of pronase in about 2 to 60 mL of water is used. If so, mucosal fluid can be immediately removed.

In order to simply administer pronase in daily practice of endoscopic examination, pronase and sodium hydrogen carbonate or alkaline ionized water for endoscopic examination can be provided in the form of a kit. Furthermore, when pronase is supplementarily administered, it would be more convenient in practice if pronase is provided in the form that enables immediate dissolution. As described above, in observation by magnifying endoscopy and endocytoscopy, since a situation where pronase is topically administered under endoscopic observation sometimes occurs, it is preferable that pronase is provided in the form that enables immediately dissolution at higher concentration than oral administration. Examples of the form include a multi-chamber device containing pronase, sodium hydrogen carbonate to make a solution alkalinity and water, or pronase and alkaline ionized water. Such a multi-chamber device is preferable in view of immediate dissolution and convenience. Examples of the multi-chamber device include a multi-chamber syringe. The syringe may be filled with individual components in advance. If a double chamber syringe is employed, one of the chambers is filled with pronase and sodium hydrogen carbonate when water is used for dissolution and the other chamber may be filled with water, or one of the chambers is filled with pronase and the other chamber may be filled alkaline ionized water.

In magnifying endoscopy and endocytoscopy, qualitatively different diagnosis can be made compared to conventional endoscopic diagnosis and brings a change in therapeutic method. In order to make highly accurate diagnosis based on magnifying endoscopy and endocytoscopy, it is essential to observe a high-quality image. Different from the colon and esophagus, when stomach is stained with a dye, an image becomes obscure, with the result that accurate diagnosis cannot be made. However, if mucosal fluid produced from the stomach is removed by a proteolytic enzyme such as pronase to clean the surface of an observation area, highly accurate diagnosis can be made without staining. When diagnostic imaging is carried out by AI, if the image to be used is not clear, diagnosis cannot be made. Therefore, it is essential to use the composition for improving diagnostic accuracy according to the present invention.

**Claims**

1. A composition for improving diagnostic accuracy for use in improving diagnostic accuracy of gastroendoscopy, containing a proteolytic enzyme for use in magnifying endoscopy or endocytoscopy, as an active ingredient.

2. The composition for improving diagnostic accuracy according to claim 1, wherein the proteolytic enzyme is pronase.

3. The composition for improving diagnostic accuracy according to Claim 1 or 2, which is administered in advance of magnifying endoscopic examination or endocytoscopic examination.

4. The composition for improving diagnostic accuracy according to any one of claims 1 to 3, which is administered in advance of magnifying endoscopic examination or endocytoscopic examination and also supplementarily administered.

5. The composition for improving diagnostic accuracy according to claim 3 or 4, wherein the composition for improving diagnostic accuracy to be administered in advance is a solution for oral administration prepared by dissolving 20,000 units of pronase and 1 g of sodium hydrogen carbonate in 50 to 80 mL of water or a solution for oral administration prepared by dissolving 20,000 units of pronase in 50 to 80 mL of alkaline ionized water.

6. The composition for improving diagnostic accuracy according to claim 4, wherein the composition for improving diagnostic accuracy to be supplementarily administered is a pronase solution prepared by dissolving 20,000 units of pronase and 1 g of sodium hydrogen carbonate in 2 to 60 mL of water or a pronase solution prepared by dissolving 20,000 units of pronase in 2 to 60 mL of alkaline ionized water.

7. The composition for improving diagnostic accuracy according to claim 4 or 6, wherein the composition for improving diagnostic accuracy to be supplementarily administered is provided in the form of a double-chamber prefilled syringe consisting of one chamber filled with pronase and sodium hydrogen carbonate and the other chamber filled with water or consisting of one chamber filled with pronase and the other chamber filled with alkaline ionized water, and dissolved just before using.

8. The composition for improving diagnostic accuracy according to any one of claims 4, 6 or 7, wherein the supplementary administration is topically administration to be performed under endoscopic observation.

9. The composition for improving diagnostic accuracy according to any one of claims 1 to 8, wherein the magnifying endoscopy or endocytoscopy is endoscopy with narrow-band imaging.

10. A mucosal membrane observation kit containing pronase, sodium hydrogen carbonate and/or alkaline ionized water for endoscopic examination.

11. The mucosal membrane observation kit for supplementary administration according to claim 10, wherein the kit is provided in the form of a double-chamber prefilled syringe consisting of one chamber filled with pronase and sodium hydrogen carbonate and the other chamber filled with water or consisting of one chamber filled with pronase and the other chamber filled with alkaline ionized water; and pronase and water or alkaline ionized water are filled such that a concentration of pronase is 333 units/ml or more and 10,000 units/ml or less.

FIG.1

**ME-NBI**

a    b  Microvascular pattern    c

Regular                Irregular                Absent

d    e  Micro-surface pattern    f

Regular                Irregular                Absent

**EC-NBI**  Regular

g    h  Microvascular pattern    i

Regular                Irregular                Absent

j    k  Micro-surface pattern    l

Regular                Irregular                Absent

FIG.2

Cases in which endoscopic submucosal dissection was performed
110 cases, 114 lesions

Excluded: 8 cases, 8 lesions
· Full magnification could not be achieved with ME-NBI
· Images with the presence of mucus, extensive adherence of blood, inadequate focus, and/or halation with ME-NBI

Cases included
102 cases, 106 lesions

106 cancerous images
each for ME-NBI and EC-NBI
(total images: 212)

106 non-cancerous images
each for ME-NBI and EC-NBI
(total images: 212)

**FIG.3**

Comparison between ME-NBI and EC-NBI by total endoscopists

a Diagnostic accuracy (%) — p<0.0001

| ME-NBI | EC-NBI |
|---|---|
| 72.2 | 78.8 |
| (IQR: 67.7-75.7) | (IQR: 74.3-85.4) |
| [95%CI: 69.3-73.6] | [95%CI: 76.4-83.0] |

b Sensitivity(%) — p<0.0001

| ME-NBI | EC-NBI |
|---|---|
| 64.2 | 82.1 |
| (IQR: 54.3-76.4) | (IQR: 72.2-89.6) |
| [95%CI: 60.4-69.8] | [95%CI: 78.3-85.9] |

c Specificity(%) — p=0.2983

| ME-NBI | EC-NBI |
|---|---|
| 83.0 | 88.7 |
| (IQR: 68.4-92.0) | (IQR: 69.9-94.3) |
| [95%CI: 77.4-88.7] | [95%CI: 79.3-91.5] |

FIG.4

Comparison between ME-NBI and EC-NBI by total endoscopists

a PPV(%) — p=0.0023

| ME-NBI | EC-NBI |
|---|---|
| 78.5 | 88.7 |
| (IQR: 72.1-87.0) | (IQR: 76.8-92.6) |
| [95%CI: 75.4-85.1] | [95%CI: 82.6-90.7] |

b NPV(%) — p<0.0001

| ME-NBI | EC-NBI |
|---|---|
| 68.5 | 79.0 |
| (IQR: 64.6-75.1) | (IQR: 72.4-84.9) |
| [95%CI: 66.4-71.6] | [95%CI: 75.3-80.5] |

FIG.5

cancerous area

non-cancerous area

FIG.6

## INTERNATIONAL SEARCH REPORT

| International application No. |
|---|
| **PCT/JP2021/034040** |

| **A.** | **CLASSIFICATION OF SUBJECT MATTER** |
|---|---|

*A61K 9/08*(2006.01)i; *A61B 1/00*(2006.01)i; *A61K 47/02*(2006.01)i; *A61K 47/22*(2006.01)i; *A61K 49/00*(2006.01)i; *A61K 49/04*(2006.01)i; *A61K 38/48*(2006.01)i
FI: A61B1/00 500; A61K38/48; A61K9/08; A61K47/02; A61K47/22; A61K49/00; A61K49/04

According to International Patent Classification (IPC) or to both national classification and IPC

| **B.** | **FIELDS SEARCHED** |
|---|---|

Minimum documentation searched (classification system followed by classification symbols)

A61B 1/00, A61K 49

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | 船津和夫, プロナーゼを用いた胃内視 検査簡易前処置法の臨床的有用性の検討, HEP, 2016, vol. 43, no. 6, pp. 17-22, non-official translation (FUNATSU, Kazuo. Examination of clinical usefulness of simple pretreatment method for gastroscopy using pronase) pp. 17-20 | 1-6, 10 |
| Y | | 4-6, 8-9 |
| A | | 7, 11 |
| Y | WO 2017/200066 A1 (MIE UNIV.) 23 November 2017 (2017-11-23) paragraphs [0151]-[0160], fig. 7-9 | 4-6, 8-9 |

| ☐ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 October 2021** | **14 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2021/034040**

| Patent document cited in search report | | | Publication date (day/month/year) | Patent family member(s) | Publication date (day/month/year) |
|---|---|---|---|---|---|
| WO | 2017/200066 | A1 | 23 November 2017 | EP    3459424    A1<br>paragraphs [0151]-[0160], fig. 7-9 | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EZOE Y. et al.** *Gastroenterology,* 2011, vol. 141, 2017-2025
- **YAO K. et al.** *Endoscopy,* 2009, vol. 41, 462-467
- **KUMAGAI Y. et al.** *Endoscopy,* 2004, vol. 36, 590-594
- **INOUE H. et al.** *Gastrointest Endosc. Clin. N. Am.,* 2004, vol. 14, 589-594
- **KUMAGAI Y. et al.** *Endoscopy,* 2017, vol. 49, 176-180
- **SASAJIMA K. et al.** *Gastrointest. Endosc.,* 2006, vol. 63, 1010-1017
- **SAKO T. et al.** *Endoscopy,* 2018, vol. 50, 69-74
- **KUDO S.E. et al.** *Gastrointest. Endosc.,* 2015, vol. 82, 912-923
- **KAISE M. et al.** *Endoscopy,* 2015, vol. 47, 19-25
- **ABAD M.R.A. et al.** *Transl. Gastroenterol. Hepatol.,* 2020, vol. 5, 28
- **MUTO M. et al.** *Dig. Endosc.,* 2016, vol. 28, 379-393
- **NAKANISHI H. et al.** *Endoscopy.,* 2017, vol. 49, 957-67